# EUROPEAN PATENT APPLICATION

(11) **EP 4 527 939 A1**
(43) Date of publication of application: **26.03.2025**
(21) Application number: 23382963.9
(22) Date of filing: 22.09.2023
(51) Int. Cl.: C12P 21/06, C12P 13/04, C07K 2/00, A01N 33/04, A01P 21/00

(54) **PROCESS FOR THE PRODUCTION OF A PROTEIN HYDROLYSATE**

(71) Applicant: In Proteins For All, S.L., 41410 Carmona (Sevilla) (ES)
(72) Inventor: Caamaño Vara, Ana Rosa, Sevilla (ES); Mielgo Caamaño, Juan Ignacio, Sevilla (ES); Mielgo Iza, Iñaki, Sevilla (ES); Mielgo Caamaño, Marta, Sevilla (ES)
(74) Representative: Araujo, Daniel

(57) **Abstract**

The invention relates to a process for the production of a protein hydrolysate suitable as plant biostimulant comprising three consecutive enzymatic hydrolysis steps with specific immobilized enzymes in bioreactors with semipermeable membranes of specific size. The first hydrolysis step is performed with a subtilisin, at pH value of 8-11, and at a temperature 50-70 °C; the second hydrolysis step is performed with a *Bacillus* metalloendopeptidase, at a pH value of 4-6, and at a temperature of 30-60 °C; and the third hydrolysis step is performed with an enzyme comprising an exopeptidase, at a pH value 4-6, and at a temperature in the range 30-60 °C. The invention also relates to the protein hydrolysate obtainable with said process and to its use as plant biostimulant.

## Description

### Technical Field

The present invention relates to a process for the production of protein hydrolysates, in particular, hydrolysates derived from plant proteins.

### Technical Background

The hydrolysis of proteins is a well-known process, widely performed for the industrial production of protein hydrolysates. Protein hydrolysis involves the cleavage of peptide bonds to produce a mixture of peptides of different molecular weights, and certain amount of free amino acids.

Protein hydrolysates have been extensively used for different purposes, typically, as food additives and nutritional supplements, for medical applications, in animal feed, or for microbiological purposes (Neklyudov et al., Properties and uses of protein hydrolysates (Review), Appl. Biochem. Microbiol., 2000, 35(5), 452-459).

Another growing application of protein hydrolysates is their use as plant biostimulants. A plant biostimulant is defined as any substance or microorganism applied to plants with the aim to enhance nutrition efficiency, abiotic stress tolerance and/or crop quality traits, regardless of its nutrients content (du Jardin P., Plant biostimulants: Definition, concept, main categories and regulation, Scientia Horticulturae, 2015, 196, 3-14).

Protein hydrolysis may be performed on different protein sources, of animal or plant origin, and according to different processes, namely, by enzymatic, acid or alkali hydrolysis.

Traditionally, most protein hydrolysates for use as plant biostimulants are based on products obtained through chemical hydrolysis of proteins from animal origin (Colla et al., Protein hydrolysates as biostimulants in horticulture, Scientia Horticulturae, 2015, 196, 28-38).

Chemical hydrolysis, however, has several drawbacks, as extreme temperatures and pH conditions are required, which makes it less attractive for industrial application, due to the risks involved and for being environmentally unfriendly. Additionally, the hydrolysates obtained have also several drawbacks, as chemical hydrolysis attacks all peptide bonds of proteins, leading to a high content of free amino acids, several amino acids are destroyed in the process, and racemization of the amino acids occurs, which could result in products less effective or potentially toxic.

Enzymatic hydrolysis avoids such drawbacks, however, the production costs are high, and usually combined enzymes are required for optimal hydrolysis levels, while the selection of specific combinations and conditions for optimal production is not trivial, and frequently depends on each particular protein source.

Regarding the protein source, there is also a growing interest in developing protein hydrolysates, suitable as plant biostimulants, using plant proteins as hydrolysis substrate.

However, even if several processes have been disclosed in the art, they have been focused only on the optimization of the hydrolysis of specific plant proteins, but a general method is not available to date.

Therefore, it would be desirable to provide an industrial process for the enzymatic production of protein hydrolysates in liquid form, particularly suited for their use as biostimulants, i.e., with optimal content of free amino acids and with peptides of optimal molecular weight, and suitable to be performed on a wide range of plant proteins as starting materials.

### Object of the invention

The object of the present invention is a process for the production of a protein hydrolysate.

Another aspect of the invention is a liquid protein hydrolysate obtained with said process.

Another aspect of the invention is a dry protein hydrolysate obtained with said liquid protein hydrolysate.

Another aspect of the invention is the use of the protein hydrolysate as plant biostimulant.

### Detailed description of the invention

The object of the present invention is a process for the production of a hydrolysate of a plant protein, wherein the process comprises three consecutive enzymatic hydrolysis steps:
a) a first hydrolysis in a first reactor using an immobilized enzyme, which is subtilisin, at a pH value in the range 8-11 and at a temperature in the range 50-70 °C, and subsequent filtration through a membrane of a molecular weight cut-off of about 10 kDa;
b) a second hydrolysis of the filtrate obtained after step a) in a second reactor using an immobilized enzyme, which is a *Bacillus* metalloendopeptidase, at a pH value in the range 4-6 and at a temperature in the range 30-60 °C, and subsequent filtration through a membrane of a molecular weight cut-off of about 5 kDa; and
c) a third hydrolysis of the filtrate obtained after step b) in a third reactor using an immobilized enzyme, which comprises an exopeptidase, at a pH value in the range 4-6 and at a temperature in the range 30-60 °C, and subsequent filtration through a membrane of a molecular weight cut-off selected from the range from about 0.5 kDa to about 1 kDa.

The authors of the present invention have developed a process for the enzymatic hydrolysis of plant proteins, which is suitable to be performed on proteins from multiple plant sources, which is cost-effective as it is suitable to be performed as a continuous process, at low temperatures and reusing the enzymes, and which produces a hydrolysate with the optimal properties to be used as plant biostimulant, in particular, with optimized peptide size and content of free amino acids.

### Definitions

In general, the terms as used in this application, unless otherwise stated, shall be understood in their ordinary meaning as known in the art.

Along the present description, as well as in the claims, singular expressions, generally preceded by the articles "a", "an" or "the", are intended to include the plural forms as well, unless the context clearly indicates otherwise.

The terms "about" or "approximately" referred to amounts, as used herein, are meant to include the exact amount and also a certain deviation around the stated amount, namely of ±10%, preferably ±5%.

The numerical ranges disclosed herein are meant to include any number falling within the ranges and also the lower and upper limits.

The process of the present invention uses immobilized enzymes. As is well-known in the art, an immobilized enzyme is an enzyme with restricted mobility, typically attached to an inert, insoluble material or otherwise retained.

Using immobilized enzymes in the present process has several advantages, for example, the process is more cost-effective, as the enzymes used in each step can be reutilized, there is no need of deactivating the enzymes by heating and, moreover, the process is more suited to be performed in continuous.

The enzymes may be immobilized by using membrane reactors. By using membrane reactors with semipermeable membranes of appropriate pore size, it is possible not only to retain the enzyme inside the reactor, but also only to allow the exit of protein hydrolysate of suitable size, according to a specific molecular weight cut-off, while larger peptides are retained to further processing. One key aspect of the present invention is, therefore, the use of membranes of selected decreasing pore size in the three connected bioreactors. Surprisingly, with the sequential enzymatic hydrolysis using specific enzymes, in specific hydrolysis conditions, combined with the selected combined pore sizes of the semipermeable membranes, the hydrolysate obtained fulfils the optimal requirements to be used as biostimulant.

The membranes, as is well-known in the art, can be made of several polymeric materials, for example, polysulfone, polyethersulfone, polyvinylpyrrolidone, polyethylene glycol, polyvinylidene fluoride, polyacrylonitrile, polyvinylchloride, poly (tetrafluoroethylene), cellulose acetate, or cellulose nitrate, among others, and they are commercially available, with different pore sizes.

In each step, a suitable membrane, of suitable pore size, is selected to allow the separation of substances of molecular weight according to the required cut-off value.

Each step of the process may be performed in a standard reactor, which can also be referred to herein as a bioreactor, to denotate that in said reactor a chemical process is carried out which involves biochemically active substances, in particular biocatalysis with enzymes.

As is well-known in the art, typically, each reactor (or bioreactor) is provided with an agitation system, a thermal jacket to adjust the temperature inside the reactor, a feeding pump to introduce the initial protein slurry (in step a)) or partial hydrolysates (in steps b) and c)), as well as to introduce other substances, for example, acid or base for pH adjustment, or water to adjust the concentration of the slurry. Additionally, each reactor is typically provided with sensor probes, particularly, to control pH and temperature conditions inside the reaction mixture.

The substrate of the hydrolysis in each reactor is typically in the form of an aqueous suspension or slurry. The term slurry, as used herein, referred to this substrate, means an aqueous suspension comprising either the protein starting material in step a) or the protein partial hydrolysates in steps b) and c).

The three reactors are connected in series, in order to perform the whole hydrolysis as a continuous process. That means that the first hydrolysate leaving the first reactor after step a), is directly fed into the second reactor and, in turn, the second hydrolysate leaving the second reactor after step b) is directly fed into the third reactor.

### First hydrolysis step

The first hydrolysis step is performed with a subtilisin enzyme.

Subtilisin (EC 3.4.21.62), as is well-known in the art, is a serine endopeptidase, which can be obtained from *Bacillus subtilis* and from other *Bacillus* species. It is widely available from several commercial sources, for example, under the tradename Alcalase^{®} (Novozymes), which is subtilisin A from *Bacillus licheniformis.*

In an embodiment, the enzyme used in the first hydrolysis step a) is subtilisin A from *Bacillus licheniformis* (Alcalase^{®}).

The determination of the suitable amount of subtilisin enzyme to be used in this hydrolysis is well within the common general knowledge of the skilled person. For example, an enzyme:substrate weight ratio of from about 1:10 to about 1:10000 can be typically used, wherein the weight of substrate means dry substrate. Preferably, the enzyme:substrate weight ratio is in the range 1:10 - 1:1000, more preferably is in the range 1:25 - 1:500, and more preferably is in the range 1:50 - 1:200.

Alternatively, the ratio of enzyme to substrate can be expressed with regard to the catalytic activity of the enzyme (rather than to its mass) to weight unit of substrate, for example as enzyme units (U) /g, wherein an enzyme unit is defined as the amount of the enzyme that catalyses the conversion of 1 micromole of a given substrate to a given product per minute, under specified conditions. In particular, protease enzyme activity can be described on the basis of the determination of degradation products from a protein substrate. Different specific units are commonly used for proteases, for example, Anson Units (AU) are frequently used, wherein one AU is defined as that amount of enzyme which per minute liberates an amount of trichloroacetic acid-soluble product which gives the same colour with phenol reagent as one milli-equivalent of tyrosine.

Therefore, alternatively, an enzyme/substrate ratio of about 0.05-0.5 AUs per gram of substrate can be typically used.

The protein substrate inside the first reactor is typically in the form of a suspension (slurry), generally having a solid concentration of about 5-25 wt%, preferably 10-20 wt%. The protein slurry is preferably pre-prepared, in a separate reactor.

The pH value is kept in the range 8-11 during this hydrolysis step. Preferably, the pH is in the range 9-10.

In order to adjust the pH value to the required range, an aqueous solution of a strong inorganic base is typically added, for example, sodium hydroxide or potassium hydroxide.

The temperature is also adjusted to a value within the range 50-70 °C, preferably within the range 55-65 °C, and still more preferably to about 60 °C.

The reaction time of this first hydrolysis step is not critical and, for example, may range from about 2 to about 8 hours. Preferably, the reaction time is from about 3 to about 7 hours, and more preferably from about 4 to about 5 hours.

After the first hydrolysis step, the reaction mixture, also containing the enzyme, is filtered through a semipermeable membrane having a molecular weight cut-off of about 10 kDa. In this way, peptides lower than 10 kDa are able to pass through it, and are sent to the second bioreactor. According to the above general definitions, it is understood that the term "about 10 kDa" includes a close range of from 9 kDa to 11 kDa.

Lager proteins/peptides, as well as the enzyme, are retained, and recirculated to the first reactor. The solid concentration of the slurry in the reactor is maintained in the range as above described, taking into account both the protein source added plus the recirculated proteins.

### Second hydrolysis step

The second hydrolysis is performed with a *Bacillus* metalloendopeptidase.

*Bacillus* metalloendopeptidase (EC 3.4.24.28), is also a well-known protease, which is also known as "bacillolysin", or "neutral protease", or *"Bacillus subtilis* neutral protease", or *"Bacillus subtilis* neutral proteinase". It is a metalloendopeptidase found, for example, in *Bacillus subtilis* or *Bacillus amyloliquefaciens.* It is widely available from several commercial sources, for example, under the tradename Neutrase^{®} (Novozymes), which is a zinc metalloendopeptidase from *Bacillus amyloliquefaciens,*

In one embodiment, the enzyme used in the second hydrolysis is Neutrase^{®}.

The determination of the suitable amount of *Bacillus* metalloendopeptidase enzyme to be used in this second hydrolysis step is also well within the common general knowledge of the skilled person. For example, an enzyme:substrate weight ratio of from about 1:10 to about 1:10000 can be typically used, wherein the weight of substrate means dry substrate. Preferably, the enzyme:substrate weight ratio is in the range 1:10 - 1:1000, more preferably is in the range 1:25 - 1:500, and more preferably is in the range 1:50 - 1:200.

Alternatively, as discussed above, the ratio of enzyme to substrate can also be expressed with regard to the catalytic activity of the enzyme, for example, as Anson Units (AU) per gram of substrate. Therefore, alternatively, an enzyme/substrate ratio of about 0.01-0.1 AUs per gram of substrate can be typically used.

The substrate inside the second bioreactor is typically in the form of a suspension (slurry), generally having a solid concentration of about 5-15 wt%, preferably of about 6-12 wt%, and more preferably of about 7-10 wt%. Additional water may be added, if needed, to adjust the concentration of the substrate inside the bioreactor.

The pH value in the second bioreactor is maintained in the range 4-6 during this hydrolysis step. Preferably, the pH is maintained in the range 4.5-5.5. In order to adjust the pH to those values, aqueous solution of a strong inorganic acid may be added, if needed, for example, hydrochloric acid.

The temperature in the second bioreactor is also adjusted to a value in the range 30-60 °C, preferably in the range 40-50 °C, and more preferably to about 45 °C.

The reaction time of this second hydrolysis step is not critical and, for example, may range from about 2 to about 8 hours. Preferably, the reaction time is from about 3 to about 7 hours, and more preferably from about 4 to about 5 hours.

After the second hydrolysis step, the reaction mixture, also containing the enzyme, is filtered through a semipermeable membrane having a molecular weight cut-off of about 5 kDa. In this way, peptides of molecular weight lower than 5 kDa are able to pass through it, and are sent to the third bioreactor. According to the above general definitions, it is understood that the term "about 5 kDa" includes a close range of from 4.5 kDa to 5.5 kDa.

Lager peptides, as well as the enzyme, are retained, and recirculated to the second reactor. The solid concentration of the slurry in the second reactor is maintained in the range as above described, taking into account both the hydrolysate fed from the first bioreactor plus the recirculated hydrolysate.

### Third hydrolysis step

The third hydrolysis step is performed with an exopeptidase. Exopeptidases, also known as exoproteases or exoproteinases, are proteolytic peptidases that catalyse the cleavage of the terminal (or next to terminal) peptide bond, to release a single amino acid, a dipeptide or a tripeptide. They can be either carboxypeptidases or aminopeptidases, depending on whether the amino acid is released from the carboxy terminus (C-terminus) or the amino terminus (N-terminus), respectively.

The exopeptidase used in the third hydrolysis of the present invention can be a carboxypeptidase, an aminopeptidase, or a mixture thereof. Preferably, comprises an aminopeptidase or a mixture of an aminopeptidase and a carboxypeptidase, more preferably comprises an aminopeptidase.

Suitable exopeptidases are, among others, those classified under EC numbers 3.4.11, for example, leucyl aminopeptidase (or leucine aminopeptidase, EC 3.4.11.1), or those classified under EC numbers 3.4.14, for example, dipeptidyl-peptidase IV (EC 3.4.14.5), or those classified under the EC numbers 3.4.17, among many others. One preferred exopeptidase is leucyl aminopeptidase (EC 3.4.11.1).

Optionally, the exopeptidase used in the third hydrolysis step can be mixed with a certain amount of endopeptidase.

In an embodiment, the enzyme used in the third hydrolysis is a pre-prepared mixture of enzymes, as are commonly available commercially. For example, in an embodiment, the enzyme is Flavourzyme^{®} (Novozyme), which is a well-known enzyme mixture comprising fungal exopeptidases and endopeptidases from *Aspergillus oryzae,* including, in particular, leucyl aminopeptidase and dipeptidyl-peptidase IV, as well as neutral protease and alkaline protease, among others (Merz et al., Flavourzyme, an Enzyme Preparation with Industrial Relevance: Automated Nine-Step Purification and Partial Characterization of Eight Enzymes, J. Agric. Food Chem., 2015, 63, 5682-93).

The determination of the suitable amount of exopeptidase or exopeptidase-endopeptidase enzyme mixture to be used in this third hydrolysis step is also well within the common general knowledge of the skilled person. For example, an enzyme:substrate weight ratio of from about 1:10 to about 1:10000 can be typically used, wherein the weight of substrate means dry substrate. Preferably, the enzyme:substrate weight ratio is in the range 1:10 - 1:1000, more preferably is in the range 1:25 - 1:500, and more preferably is in the range 1:50 - 1:200.

Alternatively, as discussed above, the ratio of enzyme to substrate can also be expressed with regard to the catalytic activity of the enzyme, for example, when the exopeptidase comprises a leucine aminopeptidase, it can be expressed as Leucine Aminopeptidase Units (LAPU) per gram of substrate, wherein one LAPU is defined as the amount of enzyme causing the hydrolysis of 1 µmol *L*-Leucine-*p*-nitroanilide to *L*-Leucine and *p*-nitroaniline per minute.

Therefore, alternatively, an enzyme/substrate ratio of about 20-150 LAPUs per gram of substrate can be typically used.

The substrate inside the third bioreactor is typically in the form of a suspension (slurry), generally having a solid concentration of about 2-12 wt%, preferably 4-8 wt%, and more preferably about 6 wt%. Additional water may be added, if needed, to adjust the concentration of the substrate inside the bioreactor.

The pH value in the third bioreactor is maintained in the range 4-6 during this hydrolysis step. Preferably, the pH is maintained in the range 4.5-5.5. In order to adjust the pH to those values, aqueous solution of a strong inorganic acid may be added, if needed, for example, hydrochloric acid.

The temperature is also adjusted to a value in the range 30-60 °C, preferably in the range 40-50 °C, and more preferably to about 45 °C.

The reaction time of this second hydrolysis step is not critical and, for example, may range from about 2 to about 8 hours. Preferably, the reaction time is from about 3 to about 7 hours, and more preferably from about 4 to about 5 hours.

After the third hydrolysis step, the reaction mixture, also containing the enzyme, is filtered through a semipermeable membrane having a molecular weight cut-off selected from the range from about 0.5 kDa to about 1 kDa.

In an embodiment the semipermeable membrane has a molecular weight cut-off of about 0.5 kDa.

In an embodiment the semipermeable membrane has a molecular weight cut-off of about 1 kDa.

In this way, peptides of molecular weight lower than said cut-off value are able to pass through it, and this filtrate is collected and constitutes the protein hydrolysate of the invention.

Lager peptides, as well as the enzyme, are retained, and recirculated again to the third reactor.

### Plant protein

The protein raw material used in the present process is usually a plant protein concentrate or plant protein isolate. Said concentrate or isolate can be obtained according to standard procedures, well-known in the art, wherein, typically, plant flour, obtained by milling plant seeds or grains, is subjected to a separation process, wherein the carbohydrates are separated and the protein fraction is obtained, following know processes, for example, as disclosed in the book chapter Mondor et al., Processing Technologies to Produce Plant Protein Concentrates and Isolates, Chapter 3 in: Plant Protein Foods, Manickavavasagan A., Lim L.T., and Ali A., Editors, Springer, 2022, 61-108.

Furthermore, plant protein concentrates or isolates are also commercially available, for example, from the companies Roquette Freres S.A., Tereos, Archer Daniel Midland, Ingredion or IFF (International Flavors & Fragrances Inc.).

Typically, the protein content of the raw material is in the range 50-85 wt%.

Said plant proteins may be from different origins, including cereals, pulses, legumes, or nuts. For example, suitable sources are soybean, wheat gluten, corn gluten rice, or sunflower proteins, among others.

Before performing the first hydrolysis step, there is typically a previous step wherein a protein slurry is prepared by mixing the plant protein starting material with water, typically with distilled or demineralized water, to obtain a suspension (slurry), which will be the substrate of the first hydrolysis. The percentage of solids in this protein slurry is generally from about 5 wt% to about 25 wt%. A percentage of solids in the range 15-20 wt% is preferred.

This previous mixing step is preferably performed in a separate vessel, also provided with agitation and heating means, before being introduced into the first bioreactor.

In order to optimise the process, it is preferred to adjust the pH and temperature of this premix (protein slurry) to the conditions to be used in the first hydrolysis reaction, i.e., to a pH value in the range 8-11 and a temperature in the range 50-70 °C., as discussed above.

The mixing time of this previous step is not critical, and is adjusted to obtain a homogeneous suspension. Typically, from 5 to 25 minutes of mixing time is adequate.

### Protein hydrolysate

The filtrate collected after the third hydrolysis step constitutes the protein hydrolysate of the present invention.

Optionally, the concentration of the liquid hydrolysate thus obtained is adjusted to a suitable value, in particular, it can be concentrated using common methods, for example, by evaporation of by reverse osmosis.

The liquid hydrolysate may be sterilized, for example, by heating of by steam sterilization, namely, by treatment in a pasteurisation system for about 5 to 20 minutes, at a temperature typically in the range 70 to 80 °C and at a pressure of 0.3-0.5 bar, or at UHT (Ultra High Temperature) for about 2 to 5 seconds at a temperature in the range 115 to 135 °C and at a pressure of 1.6-3.1 bar.

In an embodiment, the liquid protein hydrolysate has a concentration of solids from about 15 wt% to about 50 wt%, preferably from about 20 wt% to about 45 wt%, and more preferably from about 25 wt% to about 40 wt%, and is further characterized in that it contains from about 20 wt% to about 30 wt% of free amino acids, wherein the percentages are expressed by weight, relative to the total weight of the liquid hydrolysate.

All peptides of the hydrolysate have a molecular weight under the cut-off size of the membrane in the third reactor. Therefore, all peptides of the hydrolysate have a molecular weight from under 1 kDa to under 0.5 kDa, depending on the cut-off size of the membrane used.

In an embodiment, the cut-off size of the membrane is 1 kDa and all peptides of the hydrolysate have a molecular weight under 1 kDa.

In an embodiment, the cut-off size of the membrane is 0.5 kDa and all peptides of the hydrolysate have a molecular weight under 0.5 kDa.

Furthermore, independently of the specific size chosen for the membrane of the third reactor, due to the specific combination of the three consecutive hydrolysis according to the present invention, most of the peptides of the obtained hydrolysate have a molecular size under 0.5 kDa. Therefore, in all cases, typically, at least 90% of the peptides, preferably at least 95%, and more preferably at least 98% of the peptides of the hydrolysate have a molecular weight under 0.5 kDa.

Another aspect of the present invention is a liquid protein hydrolysate obtainable by the process disclosed above.

In another embodiment the liquid protein hydrolysate is dried, typically, by spray drying, to obtain a dried, powder hydrolysate.

Generally, the moisture of the dried hydrolysate is in the range 5-12 wt% and the content of free amino acids typically ranges from about 60 to about 80 wt%.

Another aspect of the present invention is a dry protein hydrolysate obtainable after drying the liquid hydrolysate.

The characteristics of the protein hydrolysate of the invention make it optimal to be used in different applications, in particular, as plant biostimulant.

And still another aspect of the invention is the use of this hydrolysate as plant biostimulant.

A plant biostimulant, as is well-known in the art, is a substance which is able to stimulate natural processes in plants, to enhance nutrition uptake and efficiency, crop quality, and tolerance to abiotic stress, benefitting both plant yield and vigour.

The hydrolysate according to the present invention has an optimal composition in terms of peptide size and percentage of free amino acids to be used as plant biostimulant.

In this connection, the process for the production of a hydrolysate of a plant protein, as hereinabove described, is to be understood as a process wherein the hydrolysate produced is suitable as plant biostimulant.

The present invention can be defined according to the following embodiments:
1.- A process for the production of a hydrolysate of a plant protein, wherein the process comprises three consecutive enzymatic hydrolysis steps:
   a) a first hydrolysis in a first reactor using an immobilized enzyme, which is subtilisin, at a pH value in the range 8-11 and at a temperature in the range 50-70 °C, and subsequent filtration through a membrane of a molecular weight cut-off of about 10 kDa;
   b) a second hydrolysis of the filtrate obtained after step a) in a second reactor using an immobilized enzyme, which is a *Bacillus* metalloendopeptidase, at a pH value in the range 4-6 and at a temperature in the range 30-60 °C, and subsequent filtration through a membrane of a molecular weight cut-off of about 5 kDa; and
   c) a third hydrolysis of the filtrate obtained after step b) in a third reactor using an immobilized enzyme, which comprises an exopeptidase, at a pH value in the range 4-6 and at a temperature in the range 30-60 °C, and subsequent filtration through a membrane of a molecular weight cut-off selected from the range from about 0.5 kDa to about 1 kDa.
2.- Process according to embodiment 1, wherein the membrane in step c) has a molecular weight cut-off of about 1 kDa.
3.- Process according to embodiment 1, wherein the membrane in step c) has a molecular weight cut-off of about 0.5 kDa.
4.- Process according to any one of embodiments 1 to 3, wherein the enzyme used in step a) is subtilisin A from *Bacillus licheniformis* (Alcalase).
5.- Process according to any one of embodiments 1 to 4, wherein the enzyme:substrate weight ratio in hydrolysis a) is in the range 1:10 to 1:10000, preferably in the range 1:10 to 1:1000, more preferably in the range 1:25 to 1:500, and still more preferably in the range 1:50 to 1:200.
6.- Process according to embodiments 1 to 5, wherein the substrate of first hydrolysis a) is a slurry having a solid content of 5-25 wt%, preferably 10-20 wt%.
7.- Process according to any one of embodiments 1 to 6, wherein the pH in step a) is in the range 9-10.
8.- Process according to any one of embodiments 1 to 7, wherein the temperature in step a) is in the range 55-65 °C, and preferably is about 60 °C.
9.- Process according to any one of embodiments 1 to 8, wherein the enzyme used in step b) is a zinc metalloendopeptidase from *Bacillus amyloliquefaciens* (Neutrase).
10.- Process according to any one of embodiments 1 to 9, wherein the enzyme:substrate weight ratio in hydrolysis b) is in the range 1:10 to 1:10000, preferably in the range 1:10 to 1:1000, more preferably in the range 1:25 to 1:500, and still more preferably in the range 1:50 to 1:200.
11.- Process according to any one of embodiments 1 to 10, wherein the substrate of second hydrolysis b) is a slurry having a solid content of 5-15 wt%, preferably 6-12 wt%, and more preferably 7-10 wt%.
12.- Process according to any one of embodiments 1 to 11, wherein the pH in step b) is in the range 4.5-5.5.
13.- Process according to any one of embodiments 1 to 12, wherein the temperature in step b) is in the range 40-50 °C, and preferably is about 45 °C.
14.- Process according to any one of embodiments 1 to 13, wherein the exopeptidase in step c) is selected from a carboxypeptidase, an aminopeptidase, and a mixture thereof, preferably is selected from an aminopeptidase and a mixture of an aminopeptidase and a carboxypeptidase.
15.- Process according to any one of embodiments 1 to 14, wherein the enzyme in step c) is a mixture of an exopeptidase and an endopeptidase.
16.- Process according to any one of embodiments 1 to 15, wherein the enzyme is step c) is a mixture comprising fungal exopeptidases and endopeptidases from *Aspergillus oryzae* (Flavourzyme).
17.- Process according to any one of embodiments 1 to 16, wherein the enzyme:substrate weight ratio in hydrolysis c) is in the range 1:10 to 1:10000, preferably in the range 1:10 to 1:1000, more preferably in the range 1:25 to 1:500, and still more preferably in the range 1:50 to 1:200.
18.- Process according to any one of embodiments 1 to 17, wherein the substrate of third hydrolysis c) is a slurry having a solid content of about 2-12 wt%, preferably 4-8 wt%, and more preferably about 6 wt%.
19.- Process according to any one of embodiments 1 to 18, wherein the pH in step c) is in the range 4.5-5.5.
20.- Process according to any one of embodiments 1 to 19, wherein the temperature in step c) is in the range 40-50 °C, and preferably is about 45 °C.
21.- Process according to any one of embodiments 1 to 20, wherein the plant protein raw material used in the first hydrolysis step is a plant protein concentrate or plant protein isolate.
22.- Process according to embodiment 21, wherein the plant protein is selected from cereal protein, pulse protein, legume protein, nuts protein, and mixtures thereof, preferably is selected from wheat protein, corn protein, rice protein, and sunflower protein.
23.- Process according to any one of embodiments 1 to 22, wherein before step a) the protein raw material is mixed with water to obtain a suspension (slurry).
24.- Process according to embodiment 23, wherein the content of solids of the slurry is from about 5 wt% to about 25 wt%, preferably from about 15 wt% to about 20 wt%.
25.- Liquid protein hydrolysate obtainable by the process according to any one of embodiments 1 to 24.
26.- Liquid protein hydrolysate according to embodiment 25, wherein the concentration of solids is from about 15 wt% to about 50 wt%, preferably from about 20 wt% to about 45 wt%; and more preferably from about 25 wt% to about 40 wt%; and wherein the content of free amino acids is from about 20 wt% to about 30 wt%, wherein the percentages are expressed by weight, relative to the total weight of the liquid hydrolysate.
27.- Liquid protein hydrolysate according to embodiment 26, wherein all peptides of the hydrolysate have a molecular weight under 1 kDa, and wherein, preferably, at least 90%, preferably at least 95% and more preferably at least 98% have a molecular weight under 0.5 kDa.
28.- Liquid protein hydrolysate according to embodiment 26, wherein all peptides of the hydrolysate have a molecular weight under 0.5 kDa.
29.- Dry protein hydrolysate obtainable by spray drying the liquid hydrolysate of any one of embodiments 25 to 28.
30.- Use of the hydrolysate of any one of embodiments 25 to 29 as plant biostimulant.

### Examples

### Example 1: Preparation of a protein hydrolysate from soybean protein

Three commercial proteases were used in the process: Alcalase^{®} 2.4L, Neutrase^{®} 0.8L and Flavourzyme^{®} 1000L, all from Novozymes (Bagsvaerd, Denmark).

Three membrane glass bioreactors (Scharlab 10L) were connected in series. The first one was provided with a membrane of Sani Membranes A/S Vibrolab3500 Denmark Synder ST MAX, having a cut-off of 10 kDa, the second one was provided with a membrane Synder MT MAX, having a cut-off of 5 kDa, and the third one was provided with a membrane of Synder XT MAX, having a cut-off of 1 kDa.

Another fourth reactor, also provided with agitation and heating means, was connected to the first one and was filled with 1,5 Kg of soybean protein isolate (Supro Soy, IFF Dupont) in powder form and 8,5 Kg of de-mineralized water. The reactor was heated to a temperature of about 60 °C and the pH was adjusted to a value of 9-10, by adding a concentrate solution of NaOH, and the agitation was maintained for 10-15 minutes, to obtain a slurry of 15-20% solid content.

The obtained slurry was sent to the first bioreactor, and the enzyme Alcalase^{®} 2.4L was also added. The amount of enzyme was calculated as a ratio of 15 g per Kg of dry soybean protein isolate.

The conditions of the hydrolysis in the first bioreactor were the following: temperature 60 °C, pH 9-10. The concentration in the first reactor was maintained at about 15-20% solids, and additional demineralized water was added, if needed. The hydrolysis reaction was maintained, under agitation, for about 4 hours. Afterwards, the reaction mixture was filtered through the first membrane (10 kDa cut-off), the filtrate was sent to the second bioreactor, while the enzyme and other retained substances were recirculated to the first bioreactor.

The conditions of the hydrolysis in the second bioreactor were the following: temperature 45 °C, pH 4.5-5.5. The concentration in the second reactor was maintained at about 10% solids, and additional demineralized water was added, if needed. The hydrolysis reaction was maintained, under agitation, for about 4 hours. Afterwards, the reaction mixture was filtered through the second membrane (5 kDa cut-off), the filtrate was sent to the third bioreactor, while the enzyme and other retained substances were recirculated to the second bioreactor.

The conditions of the hydrolysis in the third bioreactor were the following: temperature 45 °C, pH 4.5-5.5. The concentration in the second reactor was maintained at about 6% solids, and additional demineralized water was added, if needed. The hydrolysis reaction was maintained, under agitation, for about 4 hours. Afterwards, the reaction mixture was filtered through the third membrane (1 kDa cut-off), the filtrate was collected, while the enzyme and other retained substances were recirculated to the third bioreactor.

The obtained liquid protein hydrolysate was concentrated by evaporation on a subsequent film evaporation.

The characteristics of the protein hydrolysate obtained after concentration were as follows:
- 35 wt% solid content
- 25 wt% free amino acids
- 30 wt% total amino acids
- 4.8-5.2 wt% nitrogen content

### Example 2: Preparation of a protein hydrolysate from Pea Protein Isolate protein

An analogous process as disclosed in Example 1 was performed using Nutralys Pea (Roquette Freres) protein concentrate as starting material.

## Claims

1. A process for the production of a hydrolysate of a plant protein, wherein the process comprises three consecutive enzymatic hydrolysis steps:
a) a first hydrolysis in a first reactor using an immobilized enzyme, which is subtilisin, at a pH value in the range 8-11 and at a temperature in the range 50-70 °C, and subsequent filtration through a membrane of a molecular weight cut-off of about 10 kDa;
b) a second hydrolysis of the filtrate obtained after step a) in a second reactor using an immobilized enzyme, which is a *Bacillus* metalloendopeptidase, at a pH value in the range 4-6 and at a temperature in the range 30-60 °C, and subsequent filtration through a membrane of a molecular weight cut-off of about 5 kDa; and
c) a third hydrolysis of the filtrate obtained after step b) in a third reactor using an immobilized enzyme, which comprises an exopeptidase, at a pH value in the range 4-6 and at a temperature in the range 30-60 °C, and subsequent filtration through a membrane of a molecular weight cut-off selected from the range from about 0.5 kDa to about 1 kDa.

2. Process according to claim 1, wherein the enzyme used in step a) is subtilisin A from *Bacillus licheniformis* (Alcalase).

3. Process according to claims 1 or 2, wherein the substrate of first hydrolysis a) is a slurry having a solid content of 5-25 wt%, preferably 10-20 wt%.

4. Process according to any one of claims 1 to 3, wherein the pH in step a) is in the range 9-10.

5. Process according to any one of claims 1 to 4, wherein the enzyme used in step b) is a zinc metalloendopeptidase from *Bacillus amyloliquefaciens* (Neutrase).

6. Process according to any one of claims 1 to 5, wherein the substrate of second hydrolysis b) is a slurry having a solid content of 5-15 wt%, preferably 6-12 wt%, and more preferably 7-10 wt%.

7. Process according to any one of claims 1 to 6, wherein the pH in step b) is in the range 4.5-5.5.

8. Process according to any one of claims 1 to 7, wherein the enzyme in step c) is a mixture of an exopeptidase and an endopeptidase, and preferably is a mixture comprising fungal exopeptidases and endopeptidases from *Aspergillus oryzae* (Flavourzyme).

9. Process according to any one of claims 1 to 8, wherein the substrate of third hydrolysis c) is a slurry having a solid content of about 2-12 wt%, preferably 4-8 wt%, and more preferably about 6 wt%.

10. Process according to any one of claims 1 to 9, wherein the pH in step c) is in the range 4.5-5.5.

11. Process according to any one of claims 1 to 10, wherein the plant protein raw material used in the first hydrolysis step is a plant protein concentrate or plant protein isolate, preferably the plant protein is selected from cereal protein, pulse protein, legume protein, nuts protein, and mixtures thereof, preferably is selected from wheat protein, corn protein, rice protein, and sunflower protein.

12. Liquid protein hydrolysate obtainable by the process according to any one of claims 1 to 11.

13. Liquid protein hydrolysate according to claim 12, wherein the concentration of solids is from about 15% to about 50%, preferably from about 20% to about 35%; and wherein the content of free amino acids is from about 20% to about 30%, expressed by weight, relative to the total weight of the liquid hydrolysate.

14. Dry protein hydrolysate obtainable by spray drying the liquid hydrolysate of claims 12 or 13.

15. Use of the hydrolysate of any one of claims 12 to 14 as plant biostimulant.
